# FASCICULE DE BREVET EUROPEEN

(11) **EP 1 743 623 B1**
(45) Date de publication et mention de la délivrance du brevet: **29.06.2016**
(21) Numéro de dépôt: 06116879.5
(22) Date de dépôt: 10.07.2006
(51) Int. Cl.: A61K 8/42, A61K 8/66, A61Q 19/00

(54) **Utilisation de composés d'urée pour favoriser la desquamation**
Verwendung von Harnstoffverbindungen zur Förderung der Desquamation
Use of urea compounds for promoting desquamation

(30) Priorité: 13.07.2005 FR 0552189
(43) Date de publication de la demande: 17.01.2007
(73) Titulaire: L'Oréal, 75008 Paris (FR)
(72) Inventeur: Bernard, Dominique, 75015, Paris (FR); Simonetti, Lucie, 94300, Vincennes (FR); Pelletier, Pascale, 92160, Antony (FR)
(74) Mandataire: Brohmi, Karim

(56) Documents cités:
- EP-A1- 1 535 607
- FR-A1- 2 816 838
- US-A1- 2003 026 794
- ANONYMOUS: "pH stability of hydroxyalkylurea in combination either with one or more of a cyclic (lactone) and/or acyclic ester" 22 décembre 2004 (2004-12-22), IP.COM JOURNAL, IP.COM INC., WEST HENRIETTA, NY, US , XP013022595 ISSN: 1533-0001 * exemple 5 * * exemple 1 *
- PONEC M ET AL: "Lack of desquamation - the Achilles heel of the reconstructed epidermis" INTERNATIONAL JOURNAL OF COSMETIC SCIENCE, vol. 24, no. 5, 14 novembre 2002 (2002-11-14), pages 263-272, XP007911695 DOI: 10.1046/j.1467-2494.2002.00150.x
- BERNARD D ET AL: "Analysis of Proteins with Caseinolytic Activity in a Human Stratum Corneum Extract Revealed aYet Unidentiéd Cysteine Protease and Identifed the So-Called Stratum Corneum Thiol Protease as Cathepsin L2" JOURNAL OF INVESTIGATIVE DERMATOLOGY, NATURE PUBLISHING GROUP, GB, vol. 120, no. 4, 1 avril 2003 (2003-04-01) , pages 592-600, XP007911638 ISSN: 0022-202X
- NIAMKE S ET AL: "Physico-chemical and immunological properties and partial amino acid sequencing of a new metalloprotease: endoprotease Thr-N" BIOCHIMICA ET BIOPHYSICA ACTA - GENERAL SUBJECTS, ELSEVIER SCIENCE PUBLISHERS, NL, vol. 1623, no. 1, 8 septembre 2003 (2003-09-08), pages 21-28, XP004453213 ISSN: 0304-4165

## Description

La présente invention se rapporte à de nouveaux agents favorisant la desquamation, et à leur utilisation dans des compositions cosmétiques ou pharmaceutiques, notamment dermatologiques. Elle concerne également des procédés cosmétiques de traitement de la peau ou de ses annexes, et des compositions permettant leur mise en oeuvre.

La peau est constituée de deux compartiments, l'un superficiel, l'épiderme, et un plus profond, le derme, qui interagissent. L'épiderme humain naturel est composé principalement de trois types de cellules qui sont les kératinocytes, très majoritaires, les mélanocytes et les cellules de Langerhans.
L'épiderme est conventionnellement divisé en une couche basale de kératinocytes constituant la couche germinative de l'épiderme, une couche dite épineuse constituée de plusieurs couches de cellules polyédriques disposées sur les couches germinatives, une à trois couches dites granuleuses constituées de cellules aplaties contenant des inclusions cytoplasmiques distinctes, les grains de kératohyaline et enfin la couche cornée (ou *stratum corneum*), constituée d'un ensemble de couches de kératinocytes au stade terminal de leur différenciation appelés cornéocytes. Les cornéocytes sont des cellules anucléées principalement composés d'une matrice fibreuse contenant des cytokératines, entourée d'une structure très résistante de 15 nm d'épaisseur, appelée enveloppe cornée ou cornifiée. L'empilement de ces cornéocytes constitue la couche cornée qui est responsable de la fonction de barrière de l'épiderme. Au cours du processus normal de desquamation, les cornéocytes les plus superficiels se détachent de la surface de l'épiderme.
Des structures intercellulaires dérivant des desmosomes, appelées cornéosomes ou cornéodesmosomes, ont été décrites dans la couche cornée. Leur importance est majeure dans la cohésion intercornéocytaire ainsi que dans le processus de desquamation. En particulier, une corrélation étroite existe entre la dissociation cellulaire et la protéolyse de certains composants cornéodesmosomaux comme la desmogléine I ou la cornéodesmosine.
Plusieurs protéases à sérine de type trypsine ou chymotrypsine semblent être impliquées dans la protéolyse des cornéodesmosomes, en particulier l'enzyme chymotrypsique de la couche cornée (stratum corneum chymotryptic enzyme).

Le derme fournit à l'épiderme un support solide. C'est également son élément nourricier. Il est principalement constitué de fibroblastes et d'une matrice extracellulaire composée majoritairement de collagène, d'élastine et d'une substance, dite substance fondamentale. On y trouve aussi des leucocytes, des mastocytes ou encore des macrophages tissulaires. Enfin, le derme est traversé par des vaisseaux sanguins et des fibres nerveuses.
La cohésion entre l'épiderme et le derme est assurée par la jonction dermo-épidermique.

L'homéostasie épidermique correspond à un équilibre entre les kératinocytes entrant en différenciation dans les couches profondes de l'épiderme et cornéocytes éliminés à la surface de l'épiderme dans un processus appelé desquamation. De façon normale, la desquamation se déroule cornéocyte par cornéocyte et reste imperceptible.

La desquamation a par le passé été associée à des actions mécaniques. Cette théorie, dite des briques et du mortier, les briques étant les cornéocytes reliés entre eux par un mortier lipidique (P. Elias, 1984) n'a été contredite que par la découverte de structures cohésives dérivant des desmosomes appelées cornéodesmosomes liées à l'enveloppe cornée et aux filaments intermédiaires de kératines (Chapman, Walsh et al. 1991; Serre, Mils et al. 1991; Walsh and Chapman 1991). Les protéines qui composent ces structures sont les substrats des enzymes de la desquamation. En effet l'apparition progressive de fragments de protéines desmosomiales au cours de la différenciation ainsi que les modifications ultrastructurales apparentes du desmosome ont conduit les chercheurs sur la piste d'enzymes responsables de ces évolutions.
Le premier, T. Egelrud (Egelrud, Hofer et al. 1988; Egelrud and Lundstrom 1990; Egelrud and Lundstrom 1991), a mis en évidence dans le stratum corneum (SC) l'existence d'activités protéasiques de type « trypsin-like » et surtout « chymotrypsin-like » associées à la desquamation. Ces études de T. Egelrud font partie des études fondatrices de toute la recherche concernant la biochimie de la desquamation. Par la suite plusieurs protéases à cystéine et à acide aspartique ont été impliquées dans ce processus en particulier la « stratum corneum thiol protease » (SCTP) ou cathepsine L2 , la « stratum corneum cathepsin L-like » (SCCL) et la cathepsine D.

Dans un certain nombre de situations, on peut souhaiter stimuler ce mécanisme de desquamation, afin de favoriser le renouvellement épidermique et améliorer l'éclat du teint, atténuer les irrégularités de surface et lisser la peau, ou pour favoriser l'action de nettoyage et l'élimination des cellules mortes à la surface du corps. Dans des états physiologiques, on constate aussi un épaississement de la couche cornée que l'on souhaite limiter, par exemple dans le cas de callosités ou après une exposition au soleil. Par ailleurs, dans certains désordres cutanés la desquamation devient visible et ce sont des squames de grandes taille comportant de nombreux cornéocytes qui sont éliminées. Les dérégulations de la desquamation sont de mieux en mieux décrites au niveau moléculaire et associées à des aspects de peau anormaux.

Les ichtyoses sont de façon générale des exemples de différenciation épidermique altérée associée à une desquamation anormale. Dans le psoriasis maladie inflammatoire de la peau et la dermatite atopique on a pu montrer également des troubles associés à une desquamation anormale. Dans le domaine de l'acné l'accumulation d'un bouchon kératinique (éliminable par des activités protéasiques) obstruant les pores est une des raisons de l'apparition des comédons. L'apparition de pellicules est un autre exemple d'un défaut de desquamation ou les squames sont anormalement visibles. La desquamation altérée qui est présente de façon générale dans les épidermes reconstruits (Vicanova, Mommaas et al. 1996) représente également un défaut protéolytique. Certaines peaux pathologiques peuvent bénéficier de traitement prodesquamants à bases de dérivés d'urée. Parmi ces pathologies citons le syndrome de Netherton, le syndrome de Papillon-Lefèvre plus généralement les ichtyoses d'origine génétique ainsi que le psoriasis ou la dermatite atopique.

Il a déjà été proposé différentes méthodes pour favoriser la desquamation, notamment par peeling ou gommage. On peut notamment utiliser l'acide rétinoïque et ses dérivés, les α-hydroxy-acides, comme l'acide lactique ou l'acide glycolique, ou les β hydroxy-acides, comme l'acide salicylique.

Toutefois les actifs proposés, même s'ils donnent de bons résultats, ont souvent une activité protéolytique et/ou kératolytique qui peut conduire à une irritation des zones où ils sont appliqués. Par ailleurs, l'ajout de protéase exogène dans des compositions peut poser des problèmes de stabilisation, d'irritation et faire courir un risque allergique aux sujets qui les appliquent.

Les propriétés prodesquamantes de l'urée sont généralement associées à ses propriétés de dénaturation des protéines (effet dit « kératolytique »). L'urée dénaturerait certains substrats cornéodesmosomiaux favorisant, entre autres, l'action de certaines protéases. En revanche cet effet n'est pas un effet durable pouvant stimuler les processus naturels de la desquamation car l'urée finit, surtout à des concentrations supérieures à 2M, par être dénaturante des protéases elles-mêmes. Il serait donc intéressant de trouver un composé capable de stimuler de façon durable les activités protéolytiques du stratum corneum.

Certains actifs cosmétiques sont capables de stimuler la dégradation des protéines du cornéodesmosome et donc la desquamation. La demande de brevet EP 852 949 (Shiseido) décrit l'utilisation comme agent desquamant de dérivés d'acides alpha aminés de type glycine, qui favorisent la dégradation de la desmogléine (protéine du cornéodesmosome).

Il existe donc toujours un besoin de disposer d'agents stimulant la desquamation de la peau ou des muqueuses dotés d'une bonne activité sans présenter les inconvénients des composés de l'état de la technique. Il existe en particulier un besoin de stimuler les processus naturels de desquamation notamment en favorisant les protéases associées à la desquamation et présentes naturellement dans la peau.
De manière inattendue, la demanderesse a mis en évidence que ces buts et d'autres sont atteints par l'utilisation de composés dérivés de l'urée.
C'est pourquoi la présente invention a pour objet l'utilisation d'au moins un composé de formule (I) suivante 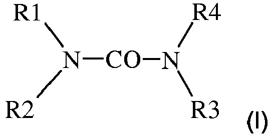 dans laquelle :
R₁, R₂, R₃ et R₄ représentent chacun, indépendamment l'un de l'autre, un atome d'hydrogène, un groupe alkyl en C₁-C₄ ou un groupe hydroxyalkyl en C₂-C₆ pouvant contenir de 1 à 5 groupes hydroxyles, où au moins un des radicaux R₁ à R₄ représente un groupe hydroxyalkyle,
ainsi que leurs sels, leurs solvats, et leurs isomères ;
dans une composition contenant un milieu physiologiquement acceptable, comme agent destiné à stimuler la desquamation de la peau et/ou des muqueuses et/ou des phanères.

En effet, il a été découvert dans le cadre de la présente invention, que de tels composés ont un effet remarquable sur plusieurs mécanismes impliqués dans la desquamation, permettant ainsi une action complète et naturelle de stimulation de ce processus, et l'application dans tous les cas où il est souhaitable de favoriser et/ou d'accélérer l'élimination des couches mortes de l'épiderme.

Cette utilisation est cosmétique et non thérapeutique, et vise à améliorer l'apparence physique et/ou l'esthétique de l'individu dont on stimule la desquamation et/ou l'exfoliation et/ou le renouvellement cellulaire.

Pour les composés de formule (I) :
- De préférence R1 désigne un groupe hydroxyalkyle en C2-C6 et R2, R3, R4 désignent, indépendamment l'un de l'autre, un atome d'hydrogène ou un groupe alkyle en C1-C4 ;
- Préférentiellement R1 désigne un groupe hydroxyalkyle en C2-C6 comprenant de 1 à 5 groupes hydroxyles, notamment 1 groupe hydroxyle, et R2, R3, R4 désignent un atome d'hydrogène ;
- Plus préférentiellement, R1 désigne un groupe hydroxyalkyle en C2-C4 comprenant 1 groupe hydroxyle et R2, R3, R4 désignent un atome d'hydrogène.

Parmi les groupes alkyle, on peut citer les groupes méthyle, éthyle, n-propyle, isopropyle, n-butyle, iso-butyle et tert-butyle.

Parmi les groupes hydroxyalkyle, on préfère ceux contenant un seul groupe hydroxyle et en particulier les groupes hydroxyéthyle, hydroxypropyle, hydroxybutyle, hydroxypentyle et hydroxyhexyle.

Parmi les sels, on peut citer les sels d'acides minéraux, tels que l'acide sulfurique, l'acide chlorhydrique, l'acide bromhydrique, l'acide iodhydrique, l'acide phosphorique, l'acide borique. On peut également citer les sels d'acides organiques, qui peuvent comporter un ou plusieurs groupes acide carboxylique, sulfonique, ou phosphonique. Il peut s'agir d'acides aliphatiques linéaires, ramifiés ou cycliques ou encore d'acides aromatiques. Ces acides peuvent comporter, en outre, un ou plusieurs hétéroatomes choisis parmi O et N, par exemple sous la forme de groupes hydroxyle. On peut notamment citer l'acide propionique, l'acide acétique, l'acide téréphtalique, l'acide citrique et l'acide tartrique.

Par solvat, on entend un mélange stoechiométrique dudit composé de formule (I) avec une ou plusieurs molécules d'eau ou de solvant organique, un tel mélange étant issu de la synthèse du composé de formule (I).

Comme composés de formule (I) préférés, on peut citer le N-(2-hydroxyéthyl)-urée ; le N-(2-hydroxypropyl)-urée , le N-(3-hydroxypropyl)-urée ; le N-(2,3-dihydroxypropyl)- urée ; le N-(2,3,4,5,6-pentahydroxyhexyl)- urée; le N-méthyl-N-(1,3,4,5,6-pentahydroxy-2-hexyl)- urée; le N-méthyl-N'-(1-hydroxy-2-méthyl-2-propyl)- urée; le N-(1-hydroxy-2-méthyl-2-propyl)- urée; le N-(1,3-dihydroxy-2-propyl)- urée; le N-(tris-hydroxyméthylméthyl)-urée ; le N-éthyl-N'-(2-hydroxyéthyl)- urée ; le N,N-bis-(2-hydroxyéthyl)- urée ; le N,N'-bis-(2-hydroxyéthyl)- urée ; le N,N-bis-(2-hydroxypropyl)- urée ; le N,N'-Bis-(2-hydroxypropyl)- urée ; le N,N-Bis-(2-hydroxyéthyl)-N'-propyl- urée ; le N,N-Bis-(2-hydroxypropyl)-N'-(2-hydroxyéthyl)- urée ; le N-tert.Butyl-N'-(2-(hydroxyéthyl)-N'-(2-(hydroxypropyl)- urée; le N-(1,3-dihydroxy-2-propyl)-N'-(2-hydroxyéthyl)- urée; le N,N-Bis-(2-hydroxyéthyl)-N',N'-dimethyl- urée ; le N,N,N',N'-tetrakis-(2-hydroxyéthyl)- urée ; le N',N'-Bis-(2-hydroxyéthyl)-N',N'-bis-(2-hydroxypropyl)- urée ; et leurs mélanges.

De préférence, le composé de formule (I) est le N-(2-hydroxyéthyl)-urée.

Les composés de formule (I) sont des composés connus et notamment décrits dans la demande DE-A-2703185. Parmi ceux-ci, le N-(2-hydroxyéthyl)-urée est en outre disponible dans le commerce, sous forme de mélange à 50% en poids dans l'eau, auprès de la société NATIONAL STARCH sous la dénomination commerciale Hydrovance^{®}. Toutefois, à la connaissance de la demanderesse, ces composés n'avaient jamais été proposée pour stimuler les activités protéolytiques et favoriser la desquamation.

La quantité de N-(2-hydroxyéthyl)-urée ou de tout autre composé de formule (I) utilisée selon l'invention dépend du résultat recherché et en particulier de la profondeur du peeling ou de l'exfoliation que l'on cherche à obtenir, qui est elle-même fonction de la condition de la peau à améliorer, Le composé de formule (I) peut notamment être présent dans la composition selon l'invention en une teneur allant de 0,01 % à 70 % en poids, par rapport au poids total de la composition, de préférence supérieure ou égale à 0,1% en poids, voire supérieure ou égale à 1%; les concentrations pourront dans des modes de réalisation varier de 0,1 % à 20 % en poids, et seront notamment inférieures ou égales à 10 % en poids, notamment pour des applications en soin quotidien.
Des concentrations plus élevées, notamment d'environ 30 à 50% seront de préférence utilisées pour des applications peelings; ces applications seront plus espacées dans le temps, par exemple hebdomadaire ou toutes les 2 semaines, dans lesquelles les composés de formule (I) seront éliminés de la surface d'application après un temps de contact, par rinçage de la surface corporelle correspondante. Le temps de contact pourra varier par exemple de 5 min à 6 heures.

Les compositions selon l'invention peuvent être des compositions cosmétiques ou pharmaceutiques, notamment des compositions dermatologiques.
Un milieu physiologiquement acceptable est selon l'invention un milieu cosmétiquement ou pharmaceutiquement acceptable compatible avec la peau, les muqueuses, les ongles et/ou les cheveux.

Les compositions selon l'invention peuvent être appliquées sur les ongles, les cheveux et plus particulièrement sur la peau et les muqueuses. Il s'agit de préférence d'un milieu cosmétiquement acceptable, c'est-à-dire qui présente une couleur, une odeur et un toucher agréable et qui ne génèrent pas d'inconfort inacceptable.
Les compositions sont de préférence des compositions ou produits cosmétique. Par "produit cosmétique", on entend notamment toute substance ou préparation destinée à être mise en contact avec les diverses parties superficielles du corps humain (épiderme, système pileux et capillaire, ongles, lèvres et organes génitaux externes) ou avec les dents et les muqueuses buccales en vue, exclusivement ou principalement, de les nettoyer, de les parfumer, d'en modifier l'aspect et/ou de corriger les odeurs corporelles et/ou de les protéger ou de les maintenir en bon état (directive cosmétique 76/768/CEE modifiée).

Trois propriétés ont donc été étudiées :
- Le pouvoir prodesquamant, par le test de dégradation de la cornéodesmosine en enceinte climatique avec des produits formulés à 5% en actif.
   Ce test permet d'évaluer la capacité d'un actif formulé à moduler la dégradation protéolytique de la cornéodesmosine.
- Le potentiel « kératolytique » :
   Le test de solubilisation des kératines (« kératolyse »): Ce test correspond à la mise en évidence des capacités dénaturantes de l'urée et de ses dérivés.
      L'effet des molécules sur la solubilisation des kératines du SC est suivi sur gel d'électrophorèse.
- Le potentiel « activateur » des protéases acides du SC : Ce test permet de montrer que dans certaines conditions, le potentiel protéolytique de certaines protéases épidermiques pouvait être augmenté.

Selon l'un des aspects de l'invention, ledit composé de formule (I) a un effet de dégradation de la cornéodesmosine, qu'il stimule.

Selon un autre aspect de l'invention, ledit composé de formule (I) a un effet kératolytique, supérieur à celui de l'urée.

Enfin, de manière avantageuse, le composé de formule (I) tel que défini précédemment, stimule l'activité des protéases du stratum corneum. Ces enzymes sont notamment choisies dans le groupe comprenant :
- Des protéases à sérine de la famille des kallikréines telles les kallikréines 5, 7 ou 14
- Des protéases à acide aspartique telles que les cathepsine D, E ou la SASPase (Locuslink 151516), ou la protéase décrite dans la demande WO 04/007548;
- Des protéases à cystéine telle que les cathepsine B, H, L , L2 , les calpaïnes ou la caspase 14 ;
- Des métalloprotéases telles que les carboxypeptidases.

En particulier, la N-(2-hydroxyéthyl)-urée à une concentration de 0,5 à 2M, et notamment de 1 M, stimule de façon prolongée la dégradation d'un substrat protéique par les protéases acides du SC, telles que la cathepsine D ou la cathepsine L2.

Les compositions selon l'invention sont notamment des compositions exfoliantes, adaptées au gommage des rugosités de la peau. Il peut aussi s'agir de compositions de peeling, permettant d'éliminer une épaisseur plus importante de couche cornée tels que les cals.

Des compositions selon l'invention sont également des compositions destinées au nettoyage de la peau et/ou du cuir chevelu, dans lesquelles l'action des agents nettoyants sera renforcée en favorisant l'élimination des cellules mortes de la surface nettoyées. Elles seront ainsi utiles comme traitement cosmétique d'appoint des états pelliculaires et desquamatifs du cuir chevelu.

Selon encore un aspect de l'invention, la composition contenant le composé de formule (I) ou ses dérivés est une composition de soin destinée à améliorer l'état de surface de la peau et/ou des muqueuses, notamment des lèvres; en particulier, ladite composition favorisera l'élimination des rugosités et/ou des peaux mortes présentes à la surface de la peau et/ou des lèvres.

L'invention concerne également l'utilisation d'au moins un composé de formule (I) ou de ses dérivés tel que défini dans ce qui précède, pour la préparation d'une composition destinée au traitement des signes associés aux désordres de la desquamation.

L'invention a ainsi pour objet l'utilisation d'au moins un composé de formule (I) pour la préparation d'une composition destinée au traitement d'au moins un trouble cutané choisi parmi l'ichtyose, l'hyperkératose, les xéroses, le psoriasis, la dermatite atopique, l'acné, les pellicules.

Les xéroses sont des troubles cutanés dans lesquels il y a dérégulation de la desquamation; on peut citer les xéroses séniles, les xéroses hivernales, les xéroses liées à l'apparition de la ménopause ou encore à l'utilisation de certains détergents.

Dans le domaine de l'acné, l'accumulation d'un bouchon kératinique obstruant les pores est une des raisons de l'apparition de comédons; celui-ci sera plus facilement éliminé, ou sa formation ralentie ou évitée, par les activités protéolytiques et desquamantes des composés selon l'invention.

Selon l'un des modes de réalisation de l'invention, les compositions sont destinées à favoriser la cicatrisation, en favorisant l'élimination des brides.

Selon un autre mode de réalisation, les composés de formule I ou les compositions les contenant telles que définis précédemment, sont utiles pour améliorer l'aspect de l'ongle en éliminant les irrégularités de surface et en les préparant à l'application d'un produit de soin ou de maquillage. La tenue de vernis à ongle sera notamment favorisée par application préalable d'un composé de formule I, qui aura une activité kératolytique et desquamante. Les composés de formule I ou les compositions le contenant favoriseront également l'élimination des cuticules qui peuvent être présentes sur le pourtour de l'ongle. Les composés de formule I pourront être appliqués dans une formulation de base au cours d'une 1^{ère} étape, puis après séchage, le vernis à ongle sera optionnellement appliqué.

D'une manière générale, toute composition de l'invention peut être ingérée, injectée ou appliquée sur la peau (sur toute zone cutanée du corps) ou sur les muqueuses (buccale, jugale, gingivale, génitale, conjonctivale).

De préférence, une composition de l'invention est appliquée sur la peau ou les muqueuses.

Selon le mode d'administration considéré, elle peut se présenter sous toutes les formes galéniques normalement utilisées.

Pour une application topique sur la peau ou les muqueuses, la composition peut avoir la forme notamment de solutions aqueuses ou huileuses ou de dispersions du type lotion ou sérum, d'émulsions de consistance liquide ou semi-liquide du type lait, obtenues par dispersion d'une phase grasse dans une phase aqueuse (H/E) ou inversement (E/H), ou de suspensions ou émulsions de consistance molle du type crème ou gel aqueux ou anhydres, ou encore de microcapsules ou microparticules, ou de dispersions vésiculaires de type ionique et/ou non ionique ou de mousses. Ces compositions sont préparées selon les méthodes usuelles.

Pour l'injection, la composition peut se présenter sous forme de lotions aqueuses, huileuses ou sous forme de sérums. Pour les yeux, elle peut se présenter sous forme de gouttes et pour l'ingestion, elle peut se présenter sous forme de capsules, de granulés, de sirops ou de comprimés.

Les quantités des différents constituants des compositions selon l'invention sont celles classiquement utilisées dans les domaines considérés.

Dans le domaine de la cosmétique, ces compositions constituent notamment des crèmes de nettoyage, de protection, de traitement ou de soin pour le visage, pour les mains, pour les pieds, pour les grands plis anatomiques ou pour le corps (par exemple crèmes de jour, crèmes de nuit, crèmes de démaquillage, crèmes de fond de teint, crèmes anti-solaires), des fonds de teint fluides, des laits de démaquillage, des laits corporels de protection ou de soin, des laits anti-solaires, des lotions, gels ou mousses pour le soin de la peau, comme des lotions de nettoyage, des lotions de bronzage artificiel, des compositions pour le bain, des compositions désodorisantes comprenant un agent bactéricide, des gels ou lotions après-rasage, des compositions contre les piqûres d'insectes, des compositions pour traiter certaines maladies de la peau comme l'eczéma, la rosacée, le psoriasis, les lichens et les prurits sévères.

Les compositions selon l'invention peuvent également consister en des préparations solides constituant des savons ou des pains de nettoyage.

Les compositions peuvent aussi être conditionnées sous forme de composition pour aérosol comprenant également un agent propulseur sous pression.

Une composition selon l'invention peut aussi être une composition pour les soins du cuir chevelu, et notamment un shampoing, une lotion de mise en plis, une lotion traitante, une crème ou un gel coiffant, de lotions restructurantes pour les cheveux, une lotion ou un gel antichute, un shampoing antiparasitaire, antipelliculaire etc.

Une composition peut aussi être à usage bucco-dentaire, par exemple une pâte dentifrice. Dans ce cas, la composition peut contenir des adjuvants et additifs usuels pour les compositions à usage buccal et notamment des agents tensioactifs, des agents épaississants, des agents humectants, des agents de polissage tels que la silice, divers ingrédients actifs comme les fluorures, en particulier le fluorure de sodium, et éventuellement des agents édulcorants comme le saccharinate de sodium.

Lorsque la composition est une émulsion, la proportion de la phase grasse peut varier d'environ 5 % à 80 % en poids, et de préférence d'environ 5 % à 50 % en poids par rapport au poids total de la composition. Les huiles, les cires, les émulsionnants et les co-émulsionnants utilisés dans la composition sous forme d'émulsion sont choisis parmi ceux classiquement utilisés dans le domaine cosmétique. L'émulsionnant et le co-émulsionnant sont présents, dans la composition, en une proportion allant de 0,3 % à 30 % en poids, et de préférence de 0,5 % à 20 % en poids par rapport au poids total de la composition. L'émulsion peut, en outre, contenir des vésicules lipidiques.
Lorsque la composition est une solution ou un gel huileux, la phase grasse peut représenter plus de 90 % du poids total de la composition.

De façon connue, la composition cosmétique peut contenir également des adjuvants habituels dans le domaine cosmétique, tels que les gélifiants hydrophiles ou lipophiles, les additifs hydrophiles ou lipophiles, les conservateurs, les antioxydants, les solvants, les parfums, les charges, les filtres, les absorbeurs d'odeurs et les matières colorantes. Les quantités de ces différents adjuvants sont celles classiquement utilisées dans le domaine cosmétique, et par exemple varient d'environ 0,01 % à 10 % du poids total de la composition. Ces adjuvants, selon leur nature, peuvent être introduits dans la phase grasse, dans la phase aqueuse et/ou dans les sphérules lipidiques.
Comme huiles ou cires utilisables dans l'invention, on peut citer les huiles minérales (huile de vaseline), les huiles végétales (fraction liquide du beurre de karité, huile de tournesol), les huiles animales (perhydrosqualène), les huiles de synthèse (huile de Purcellin), les huiles ou cires siliconées (cyclométhicone) et les huiles fluorées (perfluoropolyéthers), les cires d'abeille, de carnauba ou paraffine. On peut ajouter à ces huiles des alcools gras et des acides gras (acide stéarique). Comme émulsionnants utilisables dans l'invention, on peut citer par exemple le stéarate de glycérol, le polysorbate 60 et le mélange de PEG-6/PEG-32/Glycol Stéarate vendu sous la dénomination de Tefose^{®} 63 par la société Gattefosse.

Comme solvants utilisables dans l'invention, on peut citer les alcools inférieurs notamment l'éthanol et l'isopropanol et le propylène glycol.

Comme gélifiants hydrophiles utilisables dans l'invention, on peut citer les polymères carboxyvinyliques (carbomer^{®}), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les bentones, les sels métalliques d'acides gras comme les stéarates d'aluminium, la silice hydrophobe, l'éthylcellulose et le polyéthylène.

Les composés de formule (I) pourront être utilisés seuls ou en mélange en toute proportion.
Selon l'un des modes de réalisation de l'invention, ils sont utilisés comme seuls agents desquamants.
Selon un autre mode de réalisation de l'invention, ils sont utilisés en association avec au moins un autre agent desquamant. La composition contient alors au moins un autre agent stimulant la desquamation, différent des composés de formule (I).
Les composés de formule (I) favorisent l'activité des autres agents, dont la quantité peut ainsi être moindre dans la composition.

Par "agent desquamant", on entend tout composé capable d'agir :
- soit directement sur la desquamation en favorisant l'exfoliation, tel que les β-hydroxyacides, en particulier l'acide salicylique et ses dérivés (dont l'acide n-octanoyl 5-salicylique) ; les α-hydroxyacides, tels que les acides glycolique, citrique, lactique, tartrique, malique ou mandélique ; l'urée ; l'acide gentisique ; les oligofucoses ; l'acide cinnamique ; l'extrait de Saphora japonica ; le resvératrol et certains dérivés d'acide jasmonique ;
- soit sur les enzymes impliquées dans la desquamation ou la dégradation des cornéodesmosomes, les glycosidases, la stratum corneum chymotryptic enzym (SCCE) voire d'autres protéases (trypsine, chymotrypsine-like). On peut citer les agents chélatant des sels minéraux : l'EDTA ; l'acide N-acyl-N,N',N' éthylène diaminetriacétique ; les composés aminosulfoniques et en particulier l'acide (N-2 hydroxyéthylpiperazine-N-2-éthane) sulfonique (HEPES) ; les dérivés de l'acide 2-oxothiazolidine-4-carboxylique (procystéine) ; les dérivés d'acides alpha aminés de type glycine (tels que décrits dans EP-0 852 949, ainsi que le méthyl glycine diacétate de sodium commercialisé par BASF sous la dénomination commerciale TRILON M) ; le miel ; les dérivés de sucre tels que l'O-octanoyl-6-D-maltose et la N-acétyl glucosamine; les extraits de châtaigne tels que ceux commercialisés par la société SILAB sous la dénomination Recoverine®, les extraits de figue de barbarie, tels que ceux commercialisés sous la dénomination Exfolactive® par la société SILAB, ou la Phytosphingosine SLC® (phytosphingosine greffée par un acide salicylique) commercialisée par la société Degussa.

Des agents desquamants adaptés à l'invention peuvent notamment être choisis dans le groupe comprenant les acides sulfoniques, les chélateurs de calcium, les α-hydroxyacides tels que les acides glycolique, citrique, lactique, tartrique, malique ou mandélique ; l'acide ascorbique et ses dérivés tels que le glucoside d'ascorbyle et l'ascorbyl phosphate de magnésium ; la nicotinamide ; l'urée ; et l'acide (N-2 hydroxyéthylpiperazine-N-2-éthane) sulfonique (HEPES), les β-hydroxyacides tels que l'acide salicylique et ses dérivés, en particulier l'acide n-octanoyl 5 salicylique, les rétinoïdes tels que le rétinol et ses esters, le rétinal, l'acide rétinoïque et ses dérivés, ceux décrits dans les documents FR 2 570377, EP199636, EP325540, EP402072, les extraits de châtaigne ou de figue de barbarie, en particulier commercialisés par SILAB; des composés réducteurs tels que la cystéine ou les précurseurs de cystéine.
Des agents desquamants utilisables sont également l'acide nicotinique et ses esters et le nicotinamide, aussi appelés vitamine B3 ou vitamine PP, et l'acide ascorbique ou ses précurseurs, tels que décrits notamment dans la demande EP1529522.
En particulier, des précurseurs de cystéine pouvant être avantageusement associés aux dérivés d'urée selon l'invention sont ceux décrits dans EP 1337234. Ces dérivés de l'acide L-2-oxothiazolidine 4-carboxylique (encore appelé "procystéine") sont utiles pour favoriser la desquamation de la peau et/ou stimuler le renouvellement épidermique et répondent à la formule suivante: 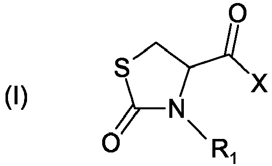 dans laquelle,
X représente un radical -OH ou un radical -NHR2, et R1, R2, identiques ou différents représentent:
   - un atome d'hydrogène,
   - un radical alkyle en C1-C8 linéaire ou ramifié, éventuellement substitué par au moins un radical -OR et/ou un radical -COOR et/ou un radical -NHR et/ou un radical -SR et/ou un radical -R pour lesquels R représente un hydrogène ou un alkyle en C1-C4 linéaire ou ramifié,
   - un radical COR₃ dans lequel R₃ représente un alkyle en C1-C8 linéaire ou ramifié, éventuellement substitué par au moins un radical -OR et/ou un radical -COOR et/ou un radical -NHR et/ou un radical -SR et/ou un radical -R pour lesquels R représente un hydrogène ou un alkyle en C1-C4 linéaire ou ramifié,
   - un radical aralkyle ou aryle benzènique ou hétérocyclique éventuellement substitué par au moins un radical -OR et/ou un radical -COOR et/ou un radical -NHR et/ou un radical -SR et/ou un radical -R pour lesquels R représente un hydrogène ou un alkyle en C1-C4 linéaire ou ramifié.

On peut citer en particulier, outre la procystéine ou acide L-2-oxo thiazolidine 4-carboxylique, l'acide 2-oxo-3-(L-γ-glutamyl) thiazolidine-4-carboxylique, la N-(2-oxothiazolidine-4-ylcarbonyl)glycine et la N-[2-oxo-3-(L-γ-glutamyl)thiazolidine-4-ylcarbonyl] glycine.

Les compositions selon l'invention pourront également contenir d'autres enzymes et en particulier des protéases exogènes, et/ou des glycosidases et/ou des lipases et/ou des amidases.

De tels composés sont classiquement utilisés dans des compositions à visée desquamante et/ou d'accélération du renouvellement de la couche cornée, et l'association à au moins un composé de formule (I) tel que défini précédemment permettra de renforcer et/ou prolonger leur action. Ces enzymes, en particulier les protéases, seront ainsi efficaces à des concentrations inférieures à celles recommandées lorsqu'ils sont utilisés dans des compositions sans agent potentialisateur, ce qui limitera les risques et difficultés liés à leur emploi.

Ces protéases peuvent être d'origine animale, végétale ou microbienne, et sont utilisées sous forme plus ou moins purifiée. On peut notamment utiliser des protéases d'origine microbienne, telles que la subtilisine, commercialisée sous la dénomination Exfocellia de COLETICA et Prozimex HTB LS 9142 de LS, ou celles disponibles dans le commerce sous des désignations commerciales Neutrase® et Esperase® vendues par la société NovoNordisk; ces protéases peuvent également être des protéases présentes dans des extraits végétaux tel que la papaye, le melon, et notamment à base de papaïne, telle que le produit Linked Papain C-PS de COLLABORATIVE LABS, de papaïne et bromélaïne, telle que le produit Vegeles LS2938 de LS, ou extraite de Mucor meihi, telle que Actizyme E3M-M de ACTIVE ORGANICS.

Les concentrations de ces enzymes seront adaptées selon le degré de pureté et l'activité du produit les contenant. Elles sont généralement de 0,0001% à 5 %, mais pourront être abaissées à des concentrations inférieures ou égales à 1%, voire inférieures ou égales à 0,1%, en association avec des composés de formule I selon l'invention.

Les glycosidases peuvent être toutes les enzymes susceptibles d'avoir comme substrat les protéoglycanes, les glycolipides et en général les glycoconjugués du stratum corneum. De telles enzymes sont notamment l'héparanase, les sialidases comme par exemple les neuraminidases, les mannosidases, les galactosidases, les glucosidases, les N-acétyl-glucosaminidases, les N-acétyl-galactosaminidases, les chondroïtinases, les glucuronidases ou encore les hyaluronidases, mais aussi les cellulases, telles que décrites par exemple dans la demande WO 02/38122; en particulier, on peut utiliser la N-glycanase, les cellulases, la β-glucosidase, la β-galactosidase, la N-acétyl-glucosaminidase ou/et la N-acétyl-galactosaminidase. Les glycosidases utilisées dans les compositions selon l'invention peuvent être purifiées à partir d'extraits de protéines synthétisées par les cellules du stratum corneum ou peuvent être des glycosidases naturellement synthétisées par des micro-organismes. Elles peuvent aussi être des glycosidases recombinantes produites en système hétérologue. Des exemples de glycosidases utilisées sont celles commercialisées par Calbiochem ou par Roche.
Il est entendu que ces enzymes peuvent être présentes dans les compositions contenant le composé de formule I seules ou en association, notamment des associations de 2 ou 3 glycosidases.

Des exemples d'amidase ou hydrolase à activité amidasique sont les composés EC 3.5.1.1 à EC 3.5.1.89 de la nomenclature classique, parmi lesquels l'asparaginase, la glutaminase, l'amidase, l'urease, l'aminoacylase, l'aspartoacylase, la ceramidase, la peptidyl-glutaminase, la formamidase, la pentanamidase, et l'aspartylglucosaminidase AGA, décrit dans la demande EP 1 438968.

Comme lipase utilisable, on peut citer de manière non limitative le produit SP144 commercialisé par la société NOVO.

Les compositions selon l'invention pourront en outre contenir au moins un actif cosmétique qui est un composé choisi parmi les agents hydratants, les agents diminuant ou inhibant l'activité des protéases néfastes, les agents stimulant la différenciation épidermique et les agents anti-séborrhéiques.

Par "agent hydratant", on entend :
- soit un composé agissant sur la fonction barrière, en vue de maintenir l'hydratation du stratum corneum, ou un composé occlusif. On peut citer les céramides, les composés à base sphingoïde, les lécithines, les glycosphingolipides, les phospholipides, le cholestérol et ses dérivés, les phytostérols (stigmastérol, β-sitostérol, campestérol), les acides gras essentiels, le 1-2 diacylglycérol, la 4-chromanone, les triterpènes pentacycliques tels que l'acide ursolique, la vaseline et la lanoline ;
- soit un composé augmentant directement la teneur en eau du stratum corneum, tel que le thréalose et ses dérivés, l'acide hyaluronique et ses dérivés, le glycérol, le pentanediol, le pidolate de sodium, la sérine, le xylitol, le lactate de sodium, le polyacrylate de glycérol, l'ectoïne et ses dérivés, le chitosane, les oligo- et polysaccharides, les carbonates cycliques, l'acide N-lauroyl pyrrolidone carboxylique, et la N-α-benzoyl-L-arginine ; l'Hepes, ou des dérivés C-glycosidés, tels que décrits dans WO 02/051828.
- soit un composé activant les glandes sébacées tel que les dérivés stéroïdiens (dont la DHEA, ses dérivés 7-oxydés et/ou 17-alkylés et les sapogénines), le dihydrojasmonate de méthyle, et la vitamine D et ses dérivés. Cette dernière catégorie de composés sera plus particulièrement adaptée aux applications sur des peaux matures, voire très matures, par exemple chez des individus ayant plus de 55 ou 60 ans.

Ces composés peuvent représenter de 0,001% à 30%, et de préférence de 0,01 à 20%, du poids total de la composition selon l'invention.

Les agents stimulant la prolifération ou la différenciation épidermique utiles dans les compositions selon l'invention sont plus spécifiquement actifs sur les kératinocytes.
Les agents stimulant la prolifération des kératinocytes, utilisables dans la composition selon l'invention, comprennent notamment les rétinoïdes tels que le rétinol et ses esters, dont le palmitate de rétinyle ; l'adénosine ; le phloroglucinol ; les extraits de tourteaux de noix commercialisés par la société GATTEFOSSE ; et les extraits de Solanum tuberosum commercialisés par la société SEDERMA.

Les agents stimulant la différenciation des kératinocytes comprennent par exemple les minéraux tels que le calcium ; un extrait peptidique de lupin tel que celui commercialisé par la société SILAB sous la dénomination commerciale Structurine^{®}; le beta-sitosteryl sulfate de sodium tel que celui commercialisé par la société SEPORGA sous la dénomination commerciale Phytocohésine^{®} ; et un extrait hydrosoluble de maïs tel que celui commercialisé par la société SOLABIA sous la dénomination commerciale Phytovityl^{®} ; un extrait peptidique de *Voandzeia substerranea* tel que celui commercialisé par la société Laboratoires Sérobiologiques sous la dénomination commerciale Filladyn LS 9397^{®} ; et les lignanes tels que le sécoisolaricirésinol.

La composition peut à ce titre également contenir de l'acide 8-hexadécène-1,16-dicarboxylique, des ecdystéroïdes tels que l'ecdystérone et ses dérivés, ou des dérivés de vitamine D, et/ou des agonistes des PPAR.

Les agents stimulant la prolifération des fibroblastes utilisables dans la composition selon l'invention peuvent par exemple être choisis parmi les protéines ou polypeptides végétaux, extraits notamment du soja (par exemple un extrait de soja commercialisé par la société LSN sous la dénomination Eleseryl SH-VEG 8® ou commercialisé par la société SILAB sous la dénomination commerciale Raffermine®) ; et les hormones végétales telles que les giberrellines et les cytokinines.

Les agents diminuant ou inhibant l'activité des protéases néfastes, on peut citer ceux agissant
- soit sur l'inhibition des métalloprotéinases (métalloprotéinases matricielles ou MMP) telles que plus particulièrement les MMP 1, 2, 3, 9. On peut citer: les rétinoïdes et dérivés, les oligopeptides et les lipopeptides, les lipoaminoacides, l'extrait de malt commercialisé par la société COLETICA sous la dénomination commerciale Collalift^{®} ; les extraits de myrtille ou de romarin ; le lycopène ; les isoflavones, leurs dérivés ou les extraits végétaux en contenant, en particulier les extraits de soja (commercialisé par exemple par la société ICHIMARU PHARCOS sous la dénomination commerciale Flavostérone SB^{®}), de trèfle rouge (commercialisé par exemple par la société SEDERMA sous la dénomination « STEROCARE^{®}» ), de lin, de kakkon ou de sauge ; les extraits de cucurma longa ; les extraits de Siegesbeckia (commercialisé par exemple par la société Sederma) ;
- soit sur l'inhibition de certaines sérine protéases telles que l'élastase leucocytaire ou la cathepsine G. On peut citer : l'extrait peptidique de graines de légumineuse (Pisum sativum) commercialisé par les Laboratoires Sérobiologiques sous la dénomination commerciale Parelastyl^{®} ; les héparinoïdes ; et les pseudodipeptides tels que l'acide {2-[acétyl-(3-trifluorométhyl-phényl)-amino]-3-méthyl-butyrylamino} acétique.

D'autres agents inhibant les protéases pouvant être associés selon l'invention sont des inhibiteurs de l'activation du plasminogène, tel que par exemple l'acide tranexamique.

En effet, si certaines activités protéasiques peuvent avantageusement être activées selon l'invention, on sait que d'autres enzymes ont un effet délétère sur les constituants du derme ou de l'épiderme et leur activité doit être réprimée pour obtenir un effet optimal sur l'aspect et l'éclat de la peau.

Selon un autre aspect, la composition contient des agents anti-séborrheiques, et par exemple un inhibiteur de 5α-réductase; ils peuvent notamment être choisis parmi :
- les rétinoïdes, et en particulier le rétinol ;
- le soufre et les dérivés soufrés ;
- les sels de zinc tels que le lactate, le gluconate, le pidolate, le carboxylate, le salicylate et/ou le cystéate de zinc ;
- le chlorure de sélénium ;
- la vitamine B6 ou pyridoxine ;
- le mélange de capryloyl glycine, de sarcosine et d'extrait de cinnamomum zeylanicum commercialisé notamment par la société SEPPIC sous la dénomination commerciale Sepicontrol A5^{®} ;
- un extrait de Laminaria saccharina commercialisé notamment par la société Biotech Marine sous la dénomination commerciale Phlorogine^{®} ;
- un extrait de Spiraea ulmaria commercialisé notamment par la société SILAB sous la dénomination commerciale Sebonormine^{®} ;
- des extraits de végétaux des espèces Arnica montana, Cinchona succirubra, Eugenia caryophyllata, Humulus lupulus, Hypericum perforatum, Mentha piperita, Rosmarinus officinalis, Salvia oficinalis et Thymus vulgaris, tous commercialisés par exemple par la société MARUZEN ;
- un extrait de Serenoa repens commercialisé notamment par la société EUROMED ;
- des extraits de plantes du genre Silybum ; et
- des extraits d'Eugenia caryophyllata contenant de l'eugénol et du glucoside d'eugényle.
Les compositions contenant de tels agents seront plus particulièrement destinées au traitement des peaux grasses ou hyperséborrhéiques.

L'invention a également pour objet un procédé de traitement cosmétique en vue de favoriser la desquamation ou pour favoriser l'élimination des couches superficielles de la peau et/ou des phanères comprenant une étape au cours de laquelle on applique sur la zone à traiter au moins un composé de formule (I), notamment le N-(2-hydroxyéthyl)-urée ou une composition le contenant.

Un autre objet de l'invention est un procédé de traitement cosmétique pour favoriser l'éclat du teint et/ou diminuer les irrégularités de surface de la peau et/ou des muqueuses, caractérisé en ce qu'on applique sur la peau ou les muqueuses au moins un composé de formule (I) ou une composition telle que décrite dans ce qui précède.
Pour la mise en oeuvre de ce procédé, le composé de formule (I) ou la composition le contenant pourront être appliqués sur toute zone de la peau ou de ses annexes, notamment du visage, du décolleté, des mains, ou sur les lèvres, afin d'atténuer les irrégularités visibles et/ou tactiles de la peau, par exemple pour atténuer les taches pigmentaires ou les cicatrices, pour lisser la surface et/ou éliminer les peaux mortes notamment des lèvres.

Les temps d'application varieront en fonction de la concentration du composé de formule (I) dans la composition, et de l'effet recherché. A titre indicatif, la composition pourra rester en contact avec la peau ou les phanères entre 5 min et 12h, elle pourra ou non être éliminée à l'issue de ce temps de contact. L'application pourra être quotidienne ou biquotidienne, ou hebdomadaire, et réitérée pendant des périodes de 2 semaines à 6 mois; cette période pouvant être prolongée ou renouvelée sans difficulté.

Selon un autre mode de réalisation, l'invention concerne un procédé cosmétique en vue de favoriser la desquamation de la peau et/ou des muqueuses, comprenant au moins une étape (i) de préparation de la peau au peeling consistant à appliquer sur les zones à traiter au moins un composé de formule (I) tel que défini dans ce qui précède, en particulier la N-(2-hydroxyéthyl)-urée, ses sels ou ses solvats, à au moins une concentration inférieure à celle entraînant la desquamation, et (ii) une étape ultérieure comprenant l'application d'au moins un agent desquamant à une concentration adaptée pour provoquer la desquamation. Une étape d'élimination du ou des agents desquamants par rinçage sera ensuite effectuée.
Avantageusement, l'étape (i) pourra être répétée avec des concentrations croissantes de composé de formule (I). Ces concentrations seront adaptées par l'homme du métier en fonction de l'effet recherché et du nombre d'applications envisagées, mais seront généralement inférieures à 10 %. On pourra par exemple utiliser une première concentration d'environ 2%, puis une ou plusieurs applications successives avec une concentration d'environ 4%, puis 6 ou 8% en poids.
L'étape (ii) comprendre l'application d'au moins un agent desquamant qui pourra être choisi dans le groupe comprenant les composés de formule (I) seuls ou en mélange, notamment la N-(2-hydroxyéthyl)-urée, mais aussi les acides sulfoniques, les chélateurs de calcium, les α-hydroxyacides tels que les acides glycolique, citrique, lactique, tartrique, malique ou mandélique ; l'acide ascorbique et ses dérivés tels que le glucoside d'ascorbyle et l'ascorbyl phosphate de magnésium ; la nicotinamide ; l'urée ; et l'acide (N-2 hydroxyéthylpiperazine-N-2-éthane) sulfonique (HEPES), les β-hydroxyacides tels que l'acide salicylique et ses dérivés, le rétinol et ses esters, le rétinal, l'acide rétinoïque et ses dérivés, les extraits de châtaigne ou de figue de barbarie, des composés réducteurs tels que la cystéine ou les précurseurs de cystéine, l'acide nicotinique et ses esters et le nicotinamide, aussi appelés vitamine B3 ou vitamine PP, l'acide 8-hexadécène-1,16-dicarboxylique.
Au cours de l'étape (ii), le (ou les) agent desquamant sera appliqué à une concentration totale supérieure ou égale à 10%, notamment d'environ 20 à 50%, et sera laissé en contact avec la peau entre 5 min et 6 heures, de préférence entre 5 min et 30 min.
Le procédé selon l'invention comprend au moins une étape supplémentaire d'élimination de l'agent desquamant, notamment du composé de formule (I) tel que la N-(2-hydroxyéthyl)-urée par rinçage à l'eau ou à l'aide d'un détergent doux.

Selon un mode de réalisation particulier l'invention concerne un procédé de traitement cosmétique des matières kératiniques qui consiste en l'application d'au moins deux composants:
- un premier composant comprenant au moins un composé de formule (I) ou une composition le contenant, tels que définis précédemment,
- un second composant comprenant au moins un agent choisi parmi les protéases, les lipases et les glycosidases,
ces deux composants étant appliqués simultanément, conjointement ou de façon séquentielle sur la peau, les muqueuses ou les phanères.
Avantageusement, le second composant comprendra au moins une protéase, impliquée dans la desquamation.
Des agents choisis parmi les agents hydratants, les agents diminuant ou inhibant l'activité des protéases néfastes, les agents stimulant la différenciation épidermique et les agents anti-séborrhéiques pourront avantageusement être présents dans le premier et/ou le second composant.

Le premier composant pourra optionnellement contenir au moins un agent desquamant, tels que ceux choisis parmi les composés favorisant directement la desquamation et les composés agissant sur les enzymes impliqués dans la desquamation ou le dégradation des cornéodesmosomes, en particulier la cystéine ou ses précurseurs.

L'invention a donc également pour objet une composition ou un agent cosmétique sous forme de kit comportant au moins deux composants
- un premier composant comprenant au moins un composé de formule (I) tel que défini précédemment,
- un second composant comprenant au moins un agent choisi parmi les protéases, les lipases et les glycosidases ,
ces deux composants étant destinés à être appliqués simultanément, conjointement ou de façon séquentielle sur la peau, les muqueuses ou les phanères.
Les agents desquamants additionnels décrits plus hauts, tels que les acides sulfoniques, les chélateurs de calcium, les α-hydroxyacides tels que les acides glycolique, citrique, lactique, tartrique, malique ou mandélique ; l'acide ascorbique et ses dérivés tels que le glucoside d'ascorbyle et l'ascorbyl phosphate de magnésium ; la nicotinamide ; l'urée ; et l'acide (N-2 hydroxyéthylpiperazine-N-2-éthane) sulfonique (HEPES), les β-hydroxyacides tels que l'acide salicylique et ses dérivés, le rétinol et ses esters, le rétinal, l'acide rétinoïque et ses dérivés, les extraits de châtaigne ou de figue de barbarie, des composés réducteurs tels que la cystéine ou les précurseurs de cystéine, l'acide nicotinique et ses esters et le nicotinamide, aussi appelés vitamine B3 ou vitamine PP, l'acide 8-hexadécène-1,16-dicarboxylique pourront également être présents dans le premier et/ou second composant.
Le premier et/ou le second composant peuvent comprendre en outre au moins un actif cosmétique tel que décrit dans ce qui précède, par exemple les agents hydratants, les agents diminuant ou inhibant l'activité des protéases néfastes, les agents stimulant la différenciation épidermique et les agents anti-séborrhéiques.

La composition telle que décrite précédemment et disposée à l'intérieur d'un récipient.

Le récipient peut être sous toute forme adéquate. Il peut être notamment sous forme d'un flacon, d'un tube, d'un pot, d'un étui, d'une boite, d'un sachet ou d'un boîtier.

L'élément de fermeture peut être sous forme d'un bouchon amovible, d'un couvercle, d'un opercule, d'une bande déchirable, ou d'une capsule, notamment du type comportant un corps fixé au récipient et une casquette articulée sur le corps. Il peut être également sous forme d'un élément assurant la fermeture sélective du récipient, notamment une pompe, une valve, ou un clapet.

Le récipient peut être associé à un applicateur, notamment sous forme d'une brosse comportant un arrangement de poils maintenus par un fil torsadé. Une telle brosse torsadée est décrite notamment dans le brevet US 4 887 622. Il peut être également sous forme d'un peigne comportant une pluralité d'éléments d'application, obtenus notamment de moulage. De tels peignes sont décrits par exemple dans le brevet FR 2 796 529. L'applicateur peut être sous forme d'un pinceau, tel que décrit par exemple dans le brevet FR 2 722 380. L'applicateur peut être sous forme d'un bloc de mousse ou d'élastomère, d'un feutre, ou d'une spatule. L'applicateur peut être libre (houppette ou éponge) ou solidaire d'une tige portée par l'élément de fermeture, tel que décrit par exemple dans le brevet US 5 492 426. L'applicateur peut être solidaire du récipient, tel que décrit par exemple le brevet FR 2 761 959.

Le produit peut être contenu directement dans le récipient, ou indirectement. A titre d'exemple, le produit peut être disposé sur un support imprégné, notamment sous forme d'une lingette ou d'un tampon, et disposé (à l'unité ou plusieurs) dans une boîte ou dans un sachet. Un tel support incorporant le produit est décrit par exemple dans la demande WO 01/03538.

L'élément de fermeture peut être couplé au récipient par vissage. Alternativement, le couplage entre l'élément de fermeture et le récipient se fait autrement que par vissage, notamment via un mécanisme à baïonnette, par encliquetage, serrage, soudage, collage, ou par attraction magnétique. Par "encliquetage" on entend en particulier tout système impliquant le franchissement d'un bourrelet ou d'un cordon de matière par déformation élastique d'une portion, notamment de l'élément de fermeture, puis par retour en position non contrainte élastiquement de ladite portion après le franchissement du bourrelet ou du cordon.

Le récipient peut être au moins pour partie réalisé en matériau thermoplastique. A titre d'exemples de matériaux thermoplastiques, on peut citer le polypropylène ou le polyéthylène.
Alternativement, le récipient est réalisé en matériau non thermoplastique, notamment en verre ou en métal (ou alliage).

Le récipient peut être à parois rigides ou à parois déformables, notamment sous forme d'un tube ou d'un flacon tube.

Le récipient peut comprendre des moyens destinés à provoquer ou faciliter la distribution de la composition. A titre d'exemple, le récipient peut être à parois déformables de manière à provoquer la sortie de la composition en réponse à une surpression à l'intérieur du récipient, laquelle surpression est provoquée par écrasement élastique (ou non élastique) des parois du récipient. Alternativement, notamment lorsque le produit est sous forme d'un stick, ce dernier peut être entraîné par un mécanisme à piston. Toujours dans le cas d'un stick, notamment de produit de maquillage, le récipient peut comporter un mécanisme, notamment à crémaillère, ou avec une tige filetée, ou avec une rampe hélicoïdale, et apte à déplacer un stick en direction de ladite ouverture. Un tel mécanisme est décrit par exemple dans le brevet FR 2 806 273 ou dans le brevet FR 2 775 566. Un tel mécanisme pour un produit liquide est décrit dans le brevet FR 2 727 609.

Le récipient peut être constitué d'un boîtier avec un fond délimitant au moins un logement contenant la composition, et un couvercle, notamment articulé sur le fond, et apte à recouvrir au moins en partie ledit fond. Un tel boîtier est décrit par exemple dans la demande WO 03/018423 ou dans le brevet FR 2 791 042.

Le récipient peut être équipé d'un essoreur disposé au voisinage de l'ouverture du récipient. Un tel essoreur permet d'essuyer l'applicateur et éventuellement, la tige dont il peut être solidaire. Un tel essoreur est décrit par exemple dans le brevet FR 2 792 618. La composition peut être à la pression atmosphérique à l'intérieur du récipient (à température ambiante) ou pressurisée, notamment au moyen d'un gaz propulseur (aérosol). Dans ce dernier cas, le récipient est équipé d'une valve (du type de celles utilisées pour les aérosols).

D'autres avantages de l'invention apparaîtront à la lecture des exemples qui suivent.

### Exemple 1 . Dégradation des cornéodesmosines en enceinte climatique

Les molécules sont formulées dans une base neutre Arlacel/Myrj. Les produits sont en contact avec des strippings vernis dans des conditions de température, d'hygrométrie et de temps d'incubation définis. On recherche la cornéodesmosine par immunodétection après l'incubation. Elle est étudiée sur immunoblot après séparation en électrophorèse et transfert sur membrane. Après un marquage spécifique avec l'anticorps G3619, elle est révélée par chimiluminescence.

On compare le produit Hydrovance®, qui est un mélange comprenant le N-(2-hydroxyéthyl)-urée commercialisé par la société National Starch, à l'urée et à un témoin.

| Formules | n° formule |
|---|---|
| Base Arlacel/Myrj | 657236 |
| Base + 5% Hydrovance® | 658890 |
| Base + 5% urée | 658892 |

### Mode opératoire:

Des rectangles de 3x3.5 cm2 sont découpés dans des strippings vernis effectués sur le bas de jambes peau sèche. Ils sont traités par 42 mg de produit (4 mg/cm2) chacun. Des contrôles et essais sont réalisés en parallèle. Les essais sont incubés à 37°C et 70% d'humidité relative pendant 5 jours ; Les contrôles sont conservés pendant 5 jours à -20°C. Pour chacun des échantillons, des poudres acétoniques sont réalisées et pesées. Les protéines sont extraites avec 100 ul de tampon Laemmli complet. Elles sont dosées selon la méthode de Bradford. Le taux de protéines de chaque échantillon est ajusté et permettra une comparaison directe des différents échantillons.

Les protéines sont séparées par électrophorèse SDS-Page sur gel à 12% d'acrylamide. Après transfert des protéines sur membrane de PVDF l'immunodétection est réalisée avec l'anticorps anticornéodesmosine G3619 (Guy Serre) au 1/12500 selon une technique de western-blot classique. La révélation est effectuée en chimiluminescence. Les bandes détectées par le fluorSmax (Biorad) sont quantifiées avec le logiciel quantity-one.

Cette expérience a été répétée deux fois et lors de la deuxième expérience, les immunoblots ont été réalisés en triplicate.

Les résultats sont une moyenne des 2 expériences réalisées.

### Résultats :

Les résultats sont exprimés en pourcentages de cornéodesmosine résiduelle par rapport au contrôle -80°C.

Plus le pourcentage est faible, plus la dégradation est importante et l'effet prodesquamant important.

| | moy des 2 expériences |
|---|---|
| base arlacel/Myrj | 79 |
| 5% Hydrovance | 23 |
| 5% urée | 30 |

Hydrovance® a un effet légèrement supérieur à l'urée.

### Exemple 2. Potentiel « kératolytique » sur les protéines du Stratum Corneum

L'urée est connue pour son activité dénaturante des protéines/ kératolytique. Une mise au point a été réalisée afin de mettre en évidence un effet de solubilisation des kératines et des protéines difficilement solubles du SC par les dérivés d'urée.

### Mode opératoire:

30 mg de poudres acétoniques de Stratum Corneum de bas de jambes de volontaires sont préparées et mis en contact avec 3 ml d'eau +2% CHAPS (détergent switterionic) pendant 10 min à température ambiante. Le mélange est ensuite broyé au potter et centrifugé 10 min à 15000g. Le surnageant est éliminé. Ce procédé permet d'éliminer les kératines et protéines très solubles qui interféreraient à l'interprétation des résultats. Le culot est repris dans 300µl d'eau + CHAPS et 20µl sont distribués dans 10 tubes eppendorf. Chaque molécule à étudier est préparée à 2M dans l'eau + 2% CHAPS.
200µl de solution sont mélangés aux 20µl d'extrait de poudres acétoniques. Les essais sont réalisés en duplicate. Un témoin sans actif est réalisé en parallèle. Les mélanges réactionnels sont incubés 10 min à température ambiante, ils sont broyés au potter puis incubés 50 min à température ambiante. Ils sont centrifugés 10 min à 15000g. Les surnageants sont recueillis et dilués au ¼ dans du tampon Laemmli 1X. Une électrophorèse est réalisée sur gel à 12,5% d'acrylamide suivie d'une coloration au nitrate d'argent.

### Résultats

La composition de (N-(2-hydroxyéthyl)-urée) (Hydrovance®) se distingue de l'urée par l'apparition de bandes de bas PM ; protéines mieux extraites ou fragments générés lors de l'incubation.

### Exemple 3. Potentiel activateur sur des protéases acides

### Activation de protéases acides

On évalue la propriété d'activer certaines protéases acides du SC (stratum corneum). La mesure de cette activité se fait grâce à un dosage par fluorimétrie à l'aide du kit Enzchek (Molecular Probes). Ce protocole utilise un substrat Bodipyfl-caséine qui lorsqu'il est hydrolysé libère de la fluorescence. La fluorescence libérée est directement proportionnelle à l'activité protéasique. Elle est lue directement en plaque 96 puits au spectrofluorimètre à 750V à 485 nm en excitation et 535 nm en émission.

On compare les résultats obtenus avec le N-(2-hydroxyéthyl)-urée (composé selon l'invention) à ceux de l'urée.

### Mode opératoire:

Les molécules sont préparées à 0, 1, 2 et 4 M en tampon acétate 0.1 M ; pH5.0.

Un extrait enzymatique est préparé à partir de poudres acétoniques. 2 ml de tampon PBS +0.1% de TritonX100 sont mis en contact 1 h dans de la glace pilée avec 200 mg de poudres acétoniques de Stratum Corneum. Le mélange est ensuite broyé au potter puis centrifugé 10 min à 15 000g à 4°C. Le surnageant est recueilli. Le substrat Enzchek dilué au 1/200 est incorporé dans chaque solution contenant les molécules. Les essais sont répétés trois fois.

Le mélange réactionnel est réalisé directement en plaque blanche.

10µl d'extrait enzymatique sont ajoutés à 200µl de solution contenant le substrat à 0, 1, 2 ou 4M. Les lectures sont réalisées à t0, t 2h, t 16h et t 24h.

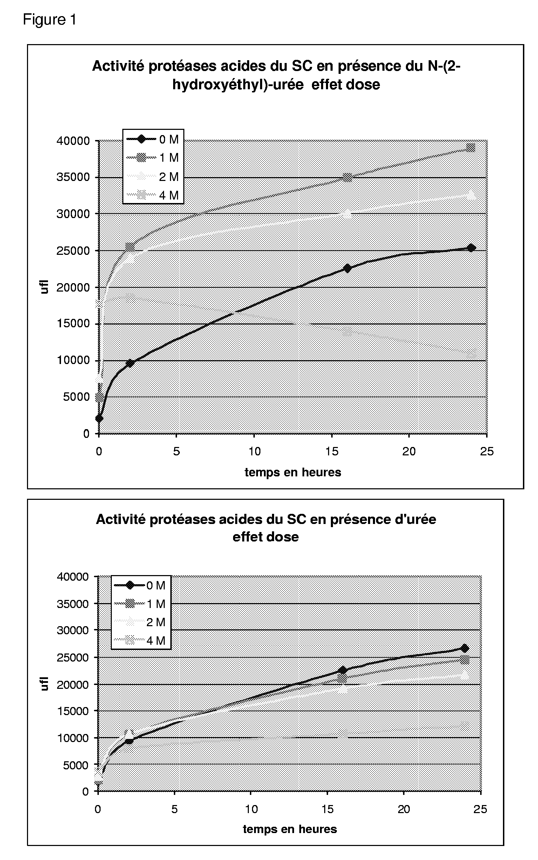

Les résultats sont représentés sur la figure en annexe

L'urée à 1 et 2M a un effet légèrement activateur sur les protéases acides jusqu'à deux heures après le début de l'incubation.

En revanche pour Hydrovance® (N-(2-hydroxyéthyl)-urée), on note une augmentation importante de l'activité pour les concentrations de 1 et 2 M par rapport au témoin. Cette augmentation est durable dans le temps. A 4M on observe une diminution nette de l'activité quel que soit le temps étudié.

### Exemple 4: Compositions

**Crème de soin:**

| | |
|---|---|
| TRIETHANOLAMINE | 0.3300 |
| WATER (and) HYDROXYETHYL UREA | 10.0000 |
| HYDROGENATED POLYISOBUTENE | 5.0000 |
| PROPYLPARABEN | 0.1000 |
| METHYLPARABEN | 0.2000 |
| CARBOMER | 0.3000 |
| CYCLOPENTASILOXANE | 15.0000 |
| WATER | 62.0700 |
| PEG-50 STEARATE | 2.5000 |
| GLYCERYL STEARATE (and) PEG-100 STEARATE | 2.5000 |
| CETYL ALCOHOL | 1.0000 |
| STEARYL ALCOHOL | 1.0000 |

La crème peut être appliquée tous les soirs sur le visage et le cou
- Formule aux esters de sucre

| | |
|---|---|
| WATER (and) HYDROXYETHYL UREA | 10.0000 |
| SHOREA ROBUSTA SEED BUTTER | 2.0000 |
| PRUNUS ARMENIACA (APRICOT) KERNEL OIL | 6.0000 |
| CHLORHEXIDINE DIGLUCONATE | 0.3000 |
| PROPYLPARABEN | 0.1000 |
| METHYLPARABEN | 0.2500 |
| XANTHAN GUM | 0.2500 |
| POLYACRYLAMIDE (and) C13-14 ISOPARAFFIN (and) LAURETH-7 | 1.0000 |
| CYCLOHEXASILOXANE | 10.0000 |
| WATER | 64.1000 |
| METHYL GLUCOSE SESQUISTEARATE | 2.0000 |
| PEG-20 METHYL GLUCOSE SESQUISTEARATE | 2.0000 |
| STEARYL ALCOHOL (and) CETEARETH-20 | 2.0000 |

La crème peut être appliquée de façon quotidienne

## Revendications

1. Utilisation cosmétique non-thérapeutique d'au moins un composé de formule (I) suivante 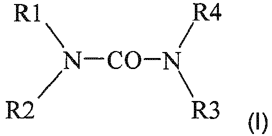 dans laquelle :
R₁, R₂, R₃ et R₄ représentent chacun, indépendamment l'un de l'autre, un atome d'hydrogène, un groupe alkyl en C₁-C₄ ou un groupe hydroxyalkyl en C₂-C₆ pouvant contenir de 1 à 5 groupes hydroxyles, où au moins un des radicaux R₁ à R₄ représente un groupe hydroxyalkyle,
ainsi que leurs sels, leurs solvats, et leurs isomères ;
dans une composition contenant un milieu physiologiquement acceptable, comme agent destiné à stimuler la desquamation de la peau et/ou des muqueuses.

2. Utilisation d'au moins un composé de formule (I) selon la revendication 1, **caractérisée en ce que** dans la formule (I) R₁ désigne un groupe hydroxyalkyle en C₂-C₆ et R₂, R₃, R₄ désignent, indépendamment l'un de l'autre, un atome d'hydrogène ou un groupe alkyle en C₁-C₄.

3. Utilisation selon l'une au moins des revendications 1 ou 2, **caractérisée en ce que** dans la formule (I) R1 désigne un groupe hydroxyalkyle en C₂-C₆ comprenant de 1 à 5 groupes hydroxyles, et R2, R3, R4 désignent un atome d'hydrogène.

4. Utilisation selon l'une au moins des revendications 1 à 3, **caractérisée en ce que** dans la formule (I) R1 désigne un groupe hydroxyalkyle en C2-C6 comprenant 1 groupe hydroxyle.

5. Utilisation selon l'une au moins des revendications 1 à 4, **caractérisée en ce que** R₁ désigne un groupe hydroxyalkyle en C2-C4 comprenant 1 groupe hydroxyle et R₂, R₃, R₄ désignent un atome d'hydrogène.

6. Utilisation selon l'une au moins des revendications 1 à 5, **caractérisé en ce que** le composé de formule (I) est choisi parmi le N-(2-hydroxyéthyl)-urée ; le N-(2-hydroxypropyl)-urée ; le N-(3-hydroxypropyl)-urée ; le N-(2,3-dihydroxypropyl)- urée ; le N-(2,3,4,5,6-pentahydroxyhexyl)- urée; le N-méthyl-N-(1,3,4,5,6-pentahydroxy-2-hexyl)-urée ; le N-méthyl-N'-(1-hydroxy-2-méthyl-2-propyl)- urée ; le N-(1-hydroxy-2-méthyl-2-propyl)- urée; le N-(1,3-dihydroxy-2-propyl)- urée; le N-(tris-hydroxyméthyl-méthyl)-urée ; le N-éthyl-N'-(2-hydroxyéthyl)- urée ; le N,N-bis-(2-hydroxyéthyl)- urée ; le N,N'-bis-(2-hydroxyéthyl)- urée ; le N,N-bis-(2-hydroxypropyl)- urée ; le N,N'-Bis-(2-hydroxypropyl)- urée ; le N,N-Bis-(2-hydroxyéthyl)-N'-propyl- urée ; le N,N-Bis-(2-hydroxypropyl)-N'-(2-hydroxyéthyl)- urée ; le N-tert.Butyl-N'-(2-(hydroxyéthyl)-N'-(2-(hydroxypropyl)- urée; le N-(1,3-dihydroxy-2-propyl)-N'-(2-hydroxyéthyl)- urée; le N,N-Bis-(2-hydroxyéthyl)-N',N'-dimethyl- urée ; le N,N,N',N'-tetrakis-(2-hydroxyéthyl)- urée ; le N',N'-Bis-(2-hydroxyéthyl)-N',N'-bis-(2-hydroxypropyl)- urée ; et leurs mélanges.

7. Utilisation selon l'une au moins des revendications 1 à 6, **caractérisée en ce que** le composé de formule (I) est le N-(2-hydroxyéthyl)-urée.

8. Utilisation d'au moins un composé de formule (I) selon l'une au moins des revendications 1 à 7, **caractérisée en ce que** ledit composé stimule la dégradation de la cornéodesmosine.

9. Utilisation selon l'une au moins des revendications 1 à 8, **caractérisé en ce que** le composé de formule (I) a une activité kératolytique.

10. Utilisation selon l'une quelconque des revendications précédentes, **caractérisé en ce que** le composé de formule (I) stimule l'activité des protéases du stratum corneum, en particulier des protéases acides.

11. Utilisation d'au moins un composé de formule (I) selon l'une au moins des revendications 1 à 10, **caractérisée en ce que** la composition est une composition exfoliante.

12. Utilisation selon l'une quelconque des revendications précédentes, **caractérisé en ce que** la composition est destinée au nettoyage de la peau et/ou du cuir chevelu.

13. Utilisation selon l'une quelconque des revendications précédentes, **caractérisé en ce que** la composition est destinée à améliorer l'état de surface de la peau et/ou des lèvres.

14. Utilisation d'au moins un composé de formule (I) tel que défini dans l'une quelconque des revendications 1 à 7, pour la préparation d'une composition destinée au traitement des signes associés aux désordres de la desquamation.

15. Utilisation selon la revendication 14, **caractérisé en ce que** la composition est destinée au traitement d'au moins un trouble cutané choisi parmi l'ichtyose, l'hyperkératose, les xéroses, le psoriasis, la dermatite atopique, l'acné, les pellicules.

16. Utilisation selon l'une des revendications 14 ou 15, **caractérisé en ce que** la composition est destinée à favoriser la cicatrisation.

17. Utilisation selon l'une au moins des revendications 1 à 16, **caractérisé en ce que** la composition est une composition cosmétique et/ou dermatologique pour application topique sur la peau et/ou les muqueuses et/ou les phanères.

18. Utilisation selon l'une quelconque des revendications 1 à 17, **caractérisée en ce que** la composition contient en outre au moins un agent stimulant la desquamation différent des composés de formule (I).

19. Utilisation selon la revendication 18, **caractérisée en ce que** l'agent stimulant la desquamation différent des composés de formule (I) est choisi dans le groupe comprenant les acides sulfoniques, les chélateurs de calcium, les α-hydroxyacides tels que les acides glycolique, citrique, lactique, tartrique, malique ou mandélique ; l'acide ascorbique et ses dérivés tels que le glucoside d'ascorbyle et l'ascorbyl phosphate de magnésium ; la nicotinamide ; l'urée ; et l'acide (N-2 hydroxyéthylpiperazine-N-2-éthane) sulfonique (HEPES), les β-hydroxyacides tels que l'acide salicylique et ses dérivés, les rétinoïdes, les extraits de châtaigne ou de figues de barbarie et des composés réducteurs tels que la cystéine ou les précurseurs de cystéine.

20. Utilisation selon l'une au moins des revendications précédentes, **caractérisée en ce que** la composition contient en outre au moins un composé choisi parmi les protéases, les glycosidases et les lipases

21. Utilisation selon l'une quelconque des revendication précédentes, **caractérisée en ce que** la composition contient en outre au moins un composé choisi parmi les agents hydratants, les agents diminuant ou inhibant l'activité des protéases néfastes, les agents stimulant la différenciation épidermique et les agents anti-séborrhéiques.

22. Procédé de traitement cosmétique non-thérapeutique pour diminuer les irrégularités de surface de la peau et/ou des muqueuses, **caractérisé en ce qu'**on applique sur la peau ou les muqueuses au moins un composé de formule (I) ou une composition le contenant selon l'une quelconque des revendications précédentes.

23. Procédé de traitement cosmétique non-thérapeutique des matières kératiniques **caractérisé en ce qu'**il consiste en l'application d'au moins deux composants:
- un premier composant comprenant au moins un composé de formule (I) selon l'une des revendications précédentes,
- un second composant comprenant au moins un agent choisi parmi les protéases, les lipases et les glycosidases,
ces deux composants étant appliqués simultanément, conjointement ou de façon séquentielle sur la peau, les muqueuses ou les phanères.

24. Procédé de traitement cosmétique selon la revendication précédente **caractérisé en ce qu'**au moins un composé choisi parmi les agents hydratants, les agents diminuant ou inhibant l'activité des protéases néfastes, les agents stimulant la différenciation épidermique, les agents anti-séborrhéiques et les agents desquamants, est en outre présent dans le premier et/ou le second composant.

25. Agent cosmétique sous forme de kit comportant au moins deux composants
- un premier composant comprenant au moins un composé de formule (I) selon l'une des revendications précédentes,
- un second composant comprenant au moins un agent choisi parmi les protéases, les lipases et les glycosidases,
ces deux composants étant appliqués simultanément, conjointement ou de façon séquentielle sur la peau, les muqueuses ou les phanères.

## Patentansprüche

1. Nichttherapeutische kosmetische Verwendung von mindestens einer Verbindung der folgenden Formel (I) 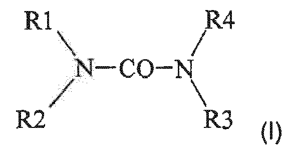 in der:
R_{1,} R₂, R₃ und R₄ jeweils unabhängig voneinander für ein Wasserstoffatom, eine C₁-C₄-Alkylgruppe oder eine C₂-C₆-Hydroxyalkylgruppe, die 1 bis 5 Hydroxylgruppen enthalten kann, stehen, wobei mindestens einer der Reste R₁ bis R₄ für eine Hydroxyalkylgruppe steht,
sowie Salzen davon, Solvaten davon und Isomeren davon
in einer Zusammensetzung, die ein physiologisch unbedenkliches Medium enthält, als Mittel zur Stimulierung der Desquamation der Haut und/oder der Schleimhäute.

2. Verwendung mindestens einer Verbindung der Formel (I) nach Anspruch 1, **dadurch gekennzeichnet, dass** in der Formel (I) R₁ für eine C₂-C₆-Hydroxyalkyl-gruppe steht und R₂, R₃ und R₄ jeweils unabhängig voneinander für ein Wasserstoffatom oder eine C₁-C₄-Alkylgruppe stehen.

3. Verwendung nach mindestens einem der Ansprüche 1 oder 2, **dadurch gekennzeichnet, dass** in der Formel (I) R₁ für eine C₂-C₆-Hydroxyalkylgruppe mit 1 bis 5 Hydroxylgruppen steht und R₂, R₃ und R₄ für ein Wasserstoffatom stehen.

4. Verwendung nach mindestens einem der Ansprüche 1 bis 3, **dadurch gekennzeichnet, dass** in der Formel (I) R₁ für eine C₂-C₆-Hydroxyalkylgruppe mit 1 Hydroxylgruppe steht.

5. Verwendung nach mindestens einem der Ansprüche 1 bis 4, **dadurch gekennzeichnet, dass** R₁ für eine C₂-C₄-Hydroxyalkylgruppe mit 1 Hydroxylgruppe steht und R₂, R₃ und R₄ für ein Wasserstoffatom stehen.

6. Verwendung nach mindestens einem der Ansprüche 1 bis 5, **dadurch gekennzeichnet, dass** die Verbindung der Formel (I) aus N-(2-Hydroxyethyl)harnstoff; N-(2-Hydroxypropyl)harnstoff; N-(3-Hydroxypropyl)-harnstoff; N-(2,3-Dihydroxypropyl)harnstoff; N-(2,3,4,5,6-Pentahydroxyhexyl)harnstoff; N-Methyl-N-(1,3,4,5,6-pentahydroxy-2-hexyl)harnstoff; N-Methyl-N'-(1-hydroxy-2-methyl-2-propyl)harn-stoff; N-(1-Hydroxy-2-methyl-2-propyl)harnstoff; N-(1,3-Dihydroxy-2-propyl)harnstoff; N-(Tris-hydroxymethylmethyl)harnstoff; N-Ethyl-N'-(2-hydroxyethyl)harnstoff; N,N-Bis(2-hydroxyethyl)-harnstoff; N,N'-Bis(2-hydroxyethyl)harnstoff; N,N-Bis(2-hydroxypropyl)harnstoff; N,N'-Bis(2-hydroxypropyl)harnstoff; N,N-Bis(2-hydroxyethyl)-N'-propylharnstoff; N,N-Bis(2-hydroxypropyl)-N'-(2-hydroxyethyl)harnstoff; N-tert-Butyl-N'-(2-hydroxyethyl)-N'-(2-hydroxypropyl)harnstoff; N-(1,3-Dihydroxy-2-propyl)-N'-(2-hydroxyethyl)-harnstoff; N,N-Bis(2-hydroxyethyl)-N',N'-dimethyl-harnstoff; N,N,N',N'-Tetrakis(2-hydroxyethyl)-harnstoff; N',N'-Bis(2-hydroxyethyl)-N',N'-bis(2-hydroxypropyl)harnstoff und Mischungen davon ausgewählt ist.

7. Verwendung nach mindestens einem der Ansprüche 1 bis 6, **dadurch gekennzeichnet, dass** es sich bei der Verbindung der Formel (I) um N-(2-Hydroxy-ethyl)harnstoff handelt.

8. Verwendung mindestens einer Verbindung der Formel (I) nach mindestens einem der Ansprüche 1 bis 7, **dadurch gekennzeichnet, dass** die Verbindung den Abbau von Corneodesmosin stimuliert.

9. Verwendung nach mindestens einem der Ansprüche 1 bis 8, **dadurch gekennzeichnet, dass** die Verbindung der Formel (I) eine keratolytische Wirkung hat.

10. Verwendung nach einem der vorhergehenden Ansprüche, **dadurch gekennzeichnet, dass** die Verbindung der Formel (I) die Aktivität von Proteasen des Stratum corneum, insbesondere sauren Proteasen, stimuliert.

11. Verwendung mindestens einer Verbindung der Formel (I) nach mindestens einem der Ansprüche 1 bis 10, **dadurch gekennzeichnet, dass** es sich bei der Zusammensetzung um eine exfolierende Zusammensetzung handelt.

12. Verwendung nach einem der vorhergehenden Ansprüche, **dadurch gekennzeichnet, dass** die Zusammensetzung zur Reinigung der Haut und/oder der Kopfhaut bestimmt ist.

13. Verwendung nach einem der vorhergehenden Ansprüche, **dadurch gekennzeichnet, dass** die Zusammensetzung zur Verbesserung des Zustands der Oberfläche der Haut und/oder der Lippen bestimmt ist.

14. Verwendung mindestens einer Verbindung der Formel (I) gemäß einem der Ansprüche 1 bis 7 zur Herstellung einer Zusammensetzung zur Behandlung der mit Desquamationsstörungen assoziierten Anzeichen.

15. Verwendung nach Anspruch 14, **dadurch gekennzeichnet, dass** die Zusammensetzung zur Behandlung mindestens einer Hautstörung, die aus Ichthyose, Hyperkeratose, Xerosen, Psoriasis, atopischer Dermatitis, Akne und Schuppen ausgewählt ist, bestimmt ist.

16. Verwendung nach Anspruch 14 oder 15, **dadurch gekennzeichnet, dass** die Zusammensetzung zur Förderung der Vernarbung bestimmt ist.

17. Verwendung nach mindestens einem der Ansprüche 1 bis 16, **dadurch gekennzeichnet, dass** es sich bei der Zusammensetzung um eine kosmetische und/oder dermatologische Zusammensetzung zum topischen Aufbringen auf die Haut und/oder die Schleimhäute und/oder die Epithelanhanggebilde handelt.

18. Verwendung nach einem der Ansprüche 1 bis 17, **dadurch gekennzeichnet, dass** die Zusammensetzung außerdem mindestens ein von den Verbindungen der Formel (I) verschiedenes desquamationsförderndes Mittel enthält.

19. Verwendung nach Anspruch 18, **dadurch gekennzeichnet, dass** das von den Verbindungen der Formel (I) verschiedene desquamationsfördernde Mittel aus der Gruppe umfassend Sulfonsäuren, Calcium-Chelatbildner, α-Hydroxysäuren wie Glykolsäure, Citronensäure, Milchsäure, Weinsäure, Äpfelsäure oder Mandelsäure; Ascorbinsäure und Derivate davon wie Ascorbylglucosid und Magnesiumascorbylphosphat; Nicotinamid; Harnstoff; (N-2-Hydroxy-ethylpiperazin-N-2-ethan)sulfonsäure (HEPES), β-Hydroxysäuren wie Salicylsäure und Derivate davon, Retinoide, Kastanien- oder Kaktusfeigen-Extrakte und reduzierend wirkende Verbindungen wie Cystein oder Cystein-Vorstufen ausgewählt ist.

20. Verwendung nach mindestens einem der vorhergehenden Ansprüche, **dadurch gekennzeichnet, dass** die Zusammensetzung außerdem mindestens eine aus Proteasen, Glycosidasen und Lipasen ausgewählte Verbindung enthält.

21. Verwendung nach einem der vorhergehenden Ansprüche, **dadurch gekennzeichnet, dass** die Zusammensetzung außerdem mindestens eine aus feuchtigkeitsspendenden Mitteln, Mitteln, die die Aktivität von schädlichen Proteasen verringern oder inhibieren, Mitteln, die die Epidermisdifferenzierung stimulieren, und Antiseborrhoika ausgewählte Verbindung enthält.

22. Verfahren zur nichttherapeutischen kosmetischen Behandlung zur Verringerung von Unregelmäßigkeiten der Oberfläche der Haut und/oder der Schleimhäute, **dadurch gekennzeichnet, dass** man auf die Haut bzw. die Schleimhäute mindestens eine Verbindung der Formel (I) oder eine diese enthaltende Zusammensetzung nach einem der vorhergehenden Ansprüche aufbringt.

23. Verfahren zur nichttherapeutischen kosmetischen Behandlung von Keratinmaterialien, **dadurch gekennzeichnet, dass** es aus dem Aufbringen von mindestens zwei Komponenten besteht:
- einer ersten Komponente, die mindestens eine Verbindung der Formel (I) nach einem der vorhergehenden Ansprüche umfasst,
- einer zweiten Komponente, die mindestens ein aus Proteasen, Lipasen und Glycosidasen ausgewähltes Mittel umfasst,
wobei die beiden Komponenten gleichzeitig, gemeinsam oder nacheinander auf die Haut, die Schleimhäute oder die Epithelanhanggebilde aufgebracht werden.

24. Verfahren zur kosmetischen Behandlung nach dem vorhergehenden Anspruch, **dadurch gekennzeichnet, dass** in der ersten und/oder der zweiten Komponente außerdem mindestens eine aus feuchtigkeitsspendenden Mitteln, Mitteln, die die Aktivität von schädlichen Proteasen verringern oder inhibieren, Mitteln, die die Epidermisdifferenzierung stimulieren, Antiseborrhoika und Desquamationsmitteln ausgewählte Verbindung vorliegt.

25. Kosmetisches Mittel in Form eines Kits mit mindestens zwei Komponenten:
- einer ersten Komponente, die mindestens eine Verbindung der Formel (I) nach einem der vorhergehenden Ansprüche umfasst,
- einer zweiten Komponente, die mindestens ein aus Proteasen, Lipasen und Glycosidasen ausgewähltes Mittel umfasst,
wobei die beiden Komponenten gleichzeitig, gemeinsam oder nacheinander auf die Haut, die Schleimhäute oder die Epithelanhanggebilde aufgebracht werden.

## Claims

1. Non-therapeutic cosmetic use of at least one compound of the following formula (I) 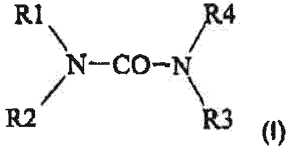 in which:
R₁, R₂, R₃ and R₄ each represent, independently of each other, a hydrogen atom, a C₁-C₄ alkyl group or a C₂-C₆ hydroxyalkyl group which may contain from 1 to 5 hydroxyl groups, where at least one of the radicals R₁ to R₄ represents a hydroxyalkyl group,
and their salts, their solvates and their isomers;
in a composition containing a physiologically acceptable medium, as agent intended for stimulating desquamation of the skin and/or the mucous membranes.

2. Use of at least one compound of formula (I) according to Claim 1, **characterized in that** in formula (I) R₁ denotes a C₂-C₆ hydroxyalkyl group and R₂, R₃ and R₄ denote, independently of each other, a hydrogen atom or a C₁-C₄ alkyl group.

3. Use according to at least either of Claims 1 and 2, **characterized in that** in formula (I) R₁ denotes a C₂-C₆ hydroxyalkyl group comprising from 1 to 5 hydroxyl groups, and R₂, R₃ and R₄ denote a hydrogen atom.

4. Use according to at least one of Claims 1 to 3, **characterized in that** in formula (I) R₁ denotes a C₂-C₆ hydroxyalkyl group comprising 1 hydroxyl group.

5. Use according to at least one of Claims 1 to 4, **characterized in that** R₁ denotes a C₂-C₄ hydroxyalkyl group comprising 1 hydroxyl group and R₂, R₃ and R₄ denote a hydrogen atom.

6. Use according to at least one of Claims 1 to 5, **characterized in that** the compound of formula (I) is chosen from N-(2-hydroxyethyl)urea; N-(2-hydroxy-propyl)urea; N-(3-hydroxypropyl)urea; N-(2,3-dihydroxypropyl)urea; N-(2,3,4,5,6-pentahydroxyhexyl)urea; N-methyl-N-(1,3,4,5,6-pentahydroxy-2-hexyl)urea; N-methyl-N'-(1-hydroxy-2-methyl-2-propyl)urea; N-(1-hydroxy-2-methyl-2-propyl)urea; N-(1,3-dihydroxy-2-propyl)urea; N-(tris-hydroxymethylmethyl)urea; N-ethyl-N'-(2-hydroxyethyl)urea; N,N-bis(2-hydroxyethyl)urea; N,N'-bis(2-hydroxyethyl)urea; N,N-bis(2-hydroxypropyl)urea; N,N'-bis(2-hydroxypropyl)urea; N,N-bis(2-hydroxyethyl)-N'-propylurea; N,N-bis(2-hydroxypropyl)-N'-(2-hydroxyethyl)urea; N-tert-butyl-N'-(2-hydroxyethyl)-N'-(2-hydroxypropyl)urea; N-(1,3-dihydroxy-2-propyl)-N'-(2-hydroxyethyl)urea; N,N-bis(2-hydroxyethyl)-N',N'-dimethylurea; N,N,N',N'-tetrakis(2-hydroxyethyl)urea; N',N'-bis(2-hydroxyethyl)-N',N'-bis(2-hydroxypropyl)-urea; and mixtures thereof.

7. Use according to at least one of Claims 1 to 6, **characterized in that** the compound of formula (I) is N-(2-hydroxyethyl)urea.

8. Use of at least one compound of formula (I) according to at least one of Claims 1 to 7, **characterized in that** the said compound stimulates the degradation of corneodesmosin.

9. Use according to at least one of Claims 1 to 8, **characterized in that** the compound of formula (I) has a keratolytic activity.

10. Use according to any one of the preceding claims, **characterized in that** the compound of formula (I) stimulates the activity of proteases of the stratum corneum, in particular acid proteases.

11. Use of at least one compound of formula (I) according to at least one of Claims 1 to 10, **characterized in that** the composition is an exfoliating composition.

12. Use according to any one of the preceding claims, **characterized in that** the composition is intended for cleansing the skin and/or the scalp.

13. Use according to any one of the preceding claims, **characterized in that** the composition is intended to improve the surface state of the skin and/or the lips.

14. Use of at least one compound of formula (I) as defined in any one of Claims 1 to 7, for the preparation of a composition intended for the treatment of the signs associated with desquamation disorders.

15. Use according to Claim 14, **characterized in that** the composition is intended for the treatment of at least one skin disorder chosen from ichthyosis, hyperkeratosis, xeroses, psoriasis, atopic dermatitis, acne and dandruff.

16. Use according to either of Claims 14 and 15, **characterized in that** the composition is intended for promoting wound healing.

17. Use according to at least one of Claims 1 to 16, **characterized in that** the composition is a cosmetic and/or dermatological composition for topical application to the skin and/or the mucous membranes and/or the superficial body growths.

18. Use according to any one of Claims 1 to 17, **characterized in that** the composition additionally contains at least one desquamation-stimulating agent different from the compounds of formula (I).

19. Use according to Claim 18, **characterized in that** the desquamation-stimulating agent different from the compounds of formula (I) is chosen from the group comprising sulphonic acids, calcium chelators, α-hydroxy acids such as glycolic, citric, lactic, tartaric, malic or mandelic acids; ascorbic acid and its derivatives such as ascorbyl glucoside and magnesium ascorbyl phosphate; nicotinamide; urea; (N-2-hydroxyethylpiperazine-N-2-ethane)sulphonic acid (HEPES), β-hydroxy acids such as salicylic acid and its derivatives, retinoids chestnut or prickly pear extracts and reducing compounds such as cysteine or cysteine precursors.

20. Use according to at least one of the preceding claims, **characterized in that** the composition additionally contains at least one compound chosen from proteases, glycosidases and lipases.

21. Use according to any one of the preceding claims, **characterized in that** the composition additionally contains at least one compound chosen from moisturizing agents, agents reducing or inhibiting the activity of harmful proteases, agents stimulating epidermal differentiation and antiseborrhoeic agents.

22. Method of non-therapeutic cosmetic treatment for reducing the surface irregularities of the skin and/or the mucous membranes, **characterized in that** at least one compound of formula (I) or a composition containing it according to any one of the preceding claims is applied to the skin or the mucous membranes.

23. Method for the non-therapeutic cosmetic treatment of keratin materials, **characterized in that** it consists in the application of at least two components:
- a first component comprising at least one compound of formula (I) according to one of the preceding claims,
- a second component comprising at least one agent chosen from proteases, lipases and glycosidases,
these two components being applied simultaneously, conjointly or sequentially to the skin, the mucous membranes or the superficial body growths.

24. Method of cosmetic treatment according to the preceding claim, **characterized in that** at least one compound chosen from moisturizing agents, agents reducing or inhibiting the activity of harmful proteases, agents stimulating epidermal differentiation, antiseborrhoeic agents and desquamating agents, is additionally present in the first and/or the second component.

25. Cosmetic agent in the form of a kit comprising at least two components
- a first component comprising at least one compound of formula (I) according to one of the preceding claims,
- a second component comprising at least one agent chosen from proteases, lipases and glycosidases,
these two components being applied simultaneously, conjointly or sequentially to the skin, the mucous membranes or the superficial body growths.
